**Europäisches Patentamt**

**European Patent Office**

**Office européen des brevets**

(19)

(11) Veröffentlichungsnummer: **0 009 094**
**B1**

(12) **EUROPÄISCHE PATENTSCHRIFT**

(45) Veröffentlichungstag der Patentschrift:
**14.04.82**

(21) Anmeldenummer: **79102584.4**

(22) Anmeldetag: **21.07.79**

(51) Int. Cl.³: **C 07 C 85/24,** C 07 C 85/26,
C 07 C 87/50

(54) **Verfahren zur Herstellung von Methylenbrücken aufweisenden Polyarylaminen.**

(30) Priorität: **12.09.78 CH 9537/78**

(43) Veröffentlichungstag der Anmeldung:
**02.04.80 Patentblatt 80/7**

(45) Bekanntmachung des Hinweises auf die Patenterteilung:
**14.04.82 Patentblatt 82/15**

(84) Benannte Vertragsstaaten:
**AT BE DE FR GB IT**

(56) Entgegenhaltungen:
**DE-A-1 642 915**
**DE-A1-2 528 694**
**DE-A1-2 648 982**
**DE-A1-2 719 996**
**DE-A1-2 748 968**
**US-A-2 938 054**

(73) Patentinhaber: **GEBRÜDER SULZER**
**AKTIENGESELLSCHAFT, Zürcherstrasse 9,**
**CH-8401 Winterthur (CH)**

(72) Erfinder: **Biller, Efim, Hochstrasse 28, CH-8044 Zürich**
**(CH)**

(74) Vertreter: **Sparing, Nikolaus, Dipl.-Ing.,**
**Rethelstrasse 123, D-4000 Düsseldorf 1 (DE)**

ACTORUM AG.

Verfahren zur Herstellung von Methylenbrücken aufweisenden Polyarylaminen

Die Erfindung betrifft ein Verfahren zur selektiven Gewinnung von Methylenbrücken aufweisenden Polyarylaminen — d.h. Mischungen aus monomeren 2-Kern-Methylendianilinen (MDA) und mehrkernigen Polyaminen (PA) —, die durch Umsetzung von Anilin und Formaldehyd in Anwesenheit von Trifluoressigsäure (TFE) und/oder mindestens einem ihrer Homologen bzw. von ihren und/oder deren Gemischen mit anderen Säuren, deren pKa-Wert kleiner als 2,5 ist, — insbesondere in Anwesenheit von einem Gemisch aus Trifluoressigsäure und Methansulfonsäure (MSS) — in hochprotonierter Lösung hergestellt worden sind. Unter dem pKa-Wert versteht man bekanntlich denjenigen pH-Wert, bei dem eine Säure zu 50% dissoziiert ist; dieser Wert ist ein Mass für die Stärke einer Säure.

Eine hochprotonierte Lösung ist eine Lösung, bei der mehr als 50% der Aminogruppen des Anilins zu Beginn der Umsetzung protoniert sind, d.h. an ihnen ein $H^+$-Ion angelagert ist. Die Protonierung der Aminogruppen ergibt sich — bei Verwendung von praktisch vollständig dissoziierten starken Säuren als Katalysatoren — aus dem molaren Mischungsverhältnis des Anilins zu dem Katalysator. Beispielsweise wird für eine 100% protonierte Lösung die Mischung von Anilin und Säuren im molaren Verhältnis 1 : 1, für eine 50% protonierte Lösung eine solche von 1 Mol Anilin zu ½ Mol Säuren erforderlich.

Die Herstellung von Methylenbrücken aufweisenden Polyarylaminen sowie die Umwandlung solcher Verbindungen in entsprechende Methylenbrücken aufweisende Polyphenyl-polyisocyanate sind allgemein bekannt. Die Polyisocyanate werden vielfach als Zwischenprodukte für die Herstellung von Polyurethanen in Form von beispielsweise harten Schäumen, gegossenen Kunststoffprodukten und Elastomeren verwendet. Weiterhin stellen in der Polyurethanchemie insbesondere jene Polyisocyanate der Diphenylmethanreihe besonders wertvolle Ausgangsmaterialien dar, die sich durch einen hohen Gehalt an 4,4'-Diisocyanatodiphenylmethan und einen geringen Gehalt an o-Isomeren, wie z.B. 2,2'- oder 2,4'-Diisocyanatodiphenylmethan, auszeichnen. Die Basis für das erwünschte 4,4'-Diisocyanatodiphenylmethan ist 4,4'-Methylendianilin (4,4'-MDA). Man ist daher im allgemeinen bestrebt, Polyarylamine herzustellen, aus denen als Hauptprodukt möglichst reines 4,4'-MDA mit möglichst grosser Ausbeute gewonnen werden kann.

Die Herstellung der Polyarylamine erfolgt dabei in bekannter Weise durch eine Kondensationsreaktion, bei der — ausgehend von Anilin und Formaldehyd in Anwesenheit von starken Säuren als Katalysatoren — als Kondensationsprodukt eine Mischung aus monomerem 2-Kern-MDA — in der Form von 4,4'-, 2,2'- und 2,4'-Isomeren — und mehrkernigen Polyaminen entsteht; bei dem direkt erreichbaren Kondensationsprodukt — d.h. ohne über mehrere Extraktions- und Destillationsstufen und/oder durch eine teilweise Rückführung des Extraktionsproduktes in die Kondensationsstufe einen erhöhten 2-Kern-Anteil und innerhalb desselben eine Erhöhung des 4,4'-Anteils zu erreichen (DE-OS 2 528 694) — beträgt das für die angestrebte Gewinnung von 4,4'-MDA günstigste Verhältnis von MDA und PA etwa 85% 2-Kern-Methylendianiline und 15% 3- und mehrkernige Polyamine. Als Säurekatalysatoren sind dabei unter anderem Perfluoressigsäuren und organische Sulfonsäuren, insbesondere Trifluoressigsäure (TFE) und Methansulfonsäure (MSS), und deren Gemische beschrieben (DE-OS 2 528 694 und 2 648 982).

Der Gehalt der bei der Anilin/Formaldehyd-Kondensation erhaltenen Polyarylamin-Gemische an Diaminen, Triaminen, Tetraminen usw. kann dabei bekanntlich beeinflusst werden durch Verändern des Verhältnisses von Anilin zu Formaldehyd und von Anilin zur Säurekatalysator-Konzentration sowie durch die Temperatur zur Zeit der Zugabe des Formaldehyds.

Andere bei der Reaktion wichtige Faktoren sind die Temperatur der Endumlagerung und die Zeitdauer der Reaktion.

Weiterhin ist der Gehalt der Polyarylamin-Gemische an 2,2'- oder 2,4'-Isomeren in erster Linie eine Funktion der Stärke und Konzentration der eingesetzten Säure-Katalysatoren. Ein hoher Protonierungsgrad (Protonierungsgrad = Prozentsatz der Gesamtmenge an Aminogruppen, die als Ammoniumgruppen vorliegen) führt im allgemeinen zu einem erhöhten Gehalt der Kondensationsprodukte an p-Isomeren, d.h. einem erhöhten 4,4'-MDA-Anteil. Nachteilig ist dabei, dass ein hoher Protonierungsgrad mit einem hohen Neutralisationsaufwand verbunden ist. Eine Neutralisation der Säure ist jedoch nicht notwendig beim Arbeiten nach dem Verfahren gemäss DE-OS 2 648 982. Dabei werden die Polyarylamine durch Austausch gegen Ausgangsanilin gewonnen.

Für die Gewinnung von 4,4'-MDA beliebiger Reinheit aus dem rohen Kondensationsprodukt sind eine Reihe Verfahren bekannt, siehe z.B. DE-OS 2 719 996 und 2 719 997, sowie den in diesen diskutierten Stand der Technik.

Bei einem dieser Verfahren wird beispielsweise die unterschiedliche Neigung zur Komplexbildung mit Phenolen zwischen 4,4'-MDA und 2,4'- bzw. 2,2'-MDA ausgenutzt; bei einem anderen Verfahren werden freie Amine und Salze des aus 4,4'-, 2,4'- und 2,2'-MDA bestehenden 2-Kern-Anteils in Gegenwart von inerten Lösungsmitteln unter Erhitzung miteinander gemischt, wobei das 4,4'-MDA quantitativ in das Salz übergeht, das ausfällt, während 2,4'- und 2,2'-MDA in Lösung bleiben. Bei einem weiteren Verfahren wird aus dem rohen Kondensationsprodukt nach Neutralisation mittels Natronlauge durch Zugabe von Salzsäure das Chlorid-Salz des 4,4'-MDA hergestellt und ausgefällt (US-PS

2 938 054). Alle diese Verfahren haben sich in der Praxis nicht durchgesetzt.

In der technischen Praxis wird daher bisher reines 4,4'-MDA ausschliesslich durch schonende fraktionierte Vakuum-Destillation gewonnen, deren Nachteile beispielsweise bereits in der DE-OS 2 719 997 diskutiert sind.

Alle diese bekannten Verfahren benötigen einen grossen zusätzlichen Aufwand an verfahrenstechnischen Anlagen, z.B. Vakuum-Destillations-Kolonnen und/oder grosse Mengen an Neutralisations-Lösungs- und Verdünnungsmitteln sowie an Energie.

Der Erfindung liegt nun die Aufgabe zugrunde, die Gewinnung von reinem 4,4'-MDA zu vereinfachen und den dafür notwendigen Aufwand zu senken. Erfindungsgemäss wird diese Aufgabe durch die im Anspruch 1 genannten Merkmale gelöst, wenn die Kondensation von Anilin und Formaldehyd in wässriger Lösung in Anwesenheit von Trifluoressigsäure und/oder mindestens einem ihrer Homologen bzw. Gemischen davon mit anderen starken Säuren, vor allem mit organischen Sulfonsäuren, insbesondere mit Methansulfonsäure (MSS), durchgeführt wird.

Zwar ist — beispielsweise aus der DE-OS 2 748 968 — bekannt, dass die Anilinsalze der Methansulfonsäure in Wasser schlecht löslich sind. Zum Beispiel kristallisiert eine 55%ige Lösung, d.h. 55 Gew.-% Anilinsalz gelöst in 45% Wasser, bereits bei 60 bis 65°C aus. Die Anilinsalze der Trifluoressigsäure dagegen sind in Wasser gut löslich.

Es ist nun aber überraschenderweise gefunden worden, dass im Gegensatz dazu die Salze der Methylendianilin-(MDA)-Isomere der Trifluoressigsäure in Wasser bei Temperaturen unter 50°C nur schlecht bzw. unlöslich sind. Dagegen sind die MDA-Salze der Methansulfonsäure (MSS) in Wasser bei Zimmertemperatur sehr gut löslich. Die MDA-Salze einer Mischung aus TFE und MSS verhalten sich ähnlich wie diejenigen reiner TFE, wobei die Kristallisationstemperatur mit zunehmendem Anteil an MSS sinkt.

Darüberhinaus ist gefunden worden, dass die Salze des 4,4'-MDA(para-Isomer) in Wasser bzw. Salz/Wasser-Gemischen schwerer löslich sind als diejenigen der o-Isomere (2,2'- und 2,4'-) und der höheren, d.h. mehrkernigen, Polyamine.

Bei der Kondensation in Anwesenheit der erwähnten Säuren erhält man homogene Lösungen, aus denen beim Abkühlen Kristalle ausgeschieden werden, die einen hohen 4,4'-MDA-Salz-Gehalt aufweisen.

Weiterhin ist gefunden worden, dass eine Zugabe von nicht protoniertem Anilin die Löslichkeit der Salze in Wasser verbessert, dagegen aber die Anilinsalze der TFE bzw. des Gemisches TFE/MSS die Löslichkeit wenig beeinflussen. Beispielsweise durch Wäsche und/oder Rekristallisation — mit bzw. aus Wasser oder Anilinsalzlösungen, die im Kondensationsprozess vorhanden sind — lässt sich auf diese Weise praktisch jede Reinheit an 4,4'-MDA erhalten.

Bei dem erfindungsgemässen Verfahren wird, wie bereits erwähnt, als einziger bzw. mindestens ein Säurekatalysator eine Perfluoressigsäure, insbesondere TFE, allein oder im Gemisch mit anderen starken Säuren, deren pKa-Wert unter 2,5 ist, eingesetzt. Beispiele für derartige, zur Gemischbildung herangezogene Säuren sind Salzsäure, Schwefelsäure, Bromwasserstoffsäure, sowie organische Sulfonsäuren, insbesondere Methansulfonsäure oder Äthansulfonsäure. Von den genannten Säuren werden die Alkansulfonsäuren bevorzugt, da ihre wässrigen Arylaminsalzlösungen die handelsüblichen Chromnickelstähle nicht angreifen.

Das neue Verfahren erlaubt weiterhin beim Arbeiten mit grösserem Anilinüberschuss und höherem Protonierungsgrad die Belastung von auf die Kondensation folgendenden Austausch- bzw. Aufarbeitungseinheiten zu verkleinern, da die Restlösung bzw. Mutterlauge die überschüssigen Anilinsalze als Hauptprodukt enthält. Weiterhin ist es vorteilhaft, dass durch die angewandte Kristallisation die Möglichkeit gegeben ist, ohne grossen Aufwand die Qualität der Fertigprodukte, d.h. insbesondere die Reinheit des 4,4'-MDA, in weiten Grenzen zu verändern und zu bestimmen. Das erfindungsgemässe Verfahren kann sowohl kontinuierlich als auch diskontinuierlich betrieben werden.

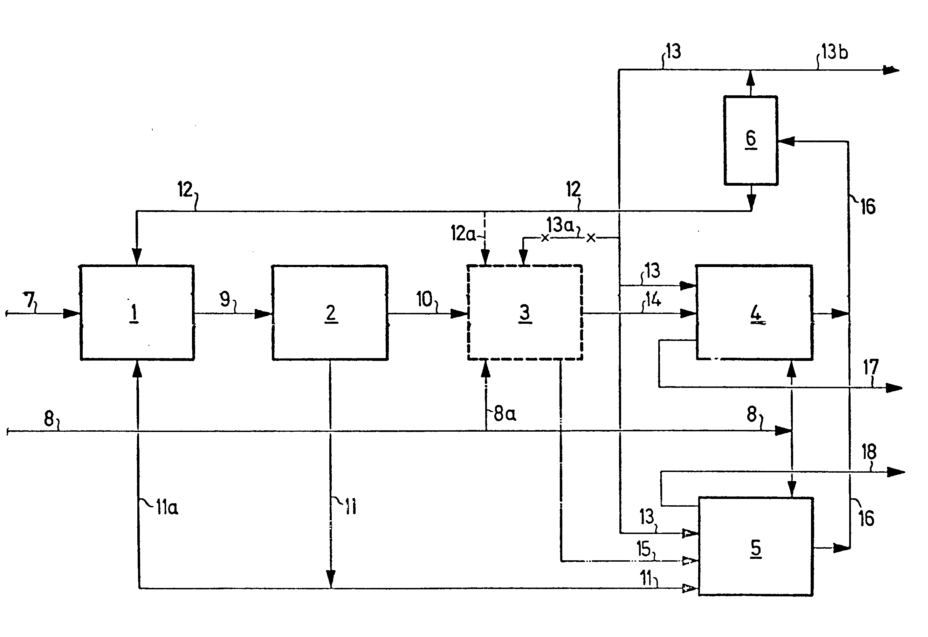

Die einzige Figur zeigt ein Verfahrensfliessbild für die Herstellung von möglichst reinem 4,4'-MDA.

Die Kondensation des Ausgangsanilins zu Di- und Polyaminen erfolgt in einem Kondensationsraum 1, dem über Strang 7 Formaldehyd und über Strang 12 eine konzentrierte wässrige Lösung von Anilinsalzen mit Säureresten eines Gemisches aus TFE und MSS zufliessen. Über Strang 11a wird in den Raum 1 unter Umständen noch Restlösung der über Strang 9 von Raum 1 nach Raum 2 fliessenden Kondensationsprodukte zurückgeführt, die nach dem erfindungsgemässen Auskristallisieren des als Produkt bevorzugten 4,4'-MDA-Salzes in Raum 2 im wesentlichen o-Isomere der MDA-Salze und die Salze der höheren Polyamine enthält. Diese Rückführung der Restlösung bezweckt, mindestens einen Teil der o-Isomere der MDA-Salze einer erneuten Kondensation mit Anilin und Formaldehyd zu unterwerfen, um den Anteil an Polyaminen zu erhöhen, die als Schaumstoff verwertbar sind.

Wie erwähnt, fliessen die Kondensationsprodukte aus Raum 1 über Strang 9 in den Raum oder die Anordnung 2, in der ein durch einfache Temperaturerniedrigung erreichtes, einstufiges oder mehrstufiges Auskristallisieren eines Teils des Kondensationsproduktes erfolgt. Die Kristalle, die zum überwiegenden Teil aus dem gewünschten 4,4'-MDA-Salz bestehen, fliessen über Strang 10 einer weiteren, nicht unbedingt notwendigen, jedoch für die Reinheit des 4,4'-Produktes vorteilhaften Behandlungsstufe 3 zu, in der — unter Zugabe von Wasser über Strang 13a, von Ausgangsanilinsalzlösung über Strang 12a und/oder von Anilin über Strang 8, 8a — eine Kristallwäsche und/oder eine Lösung und Rekristallisation der einströmenden 4,4'-MDA-Salzkristalle durchgeführt wird.

Von der Behandlungsstufe 3 werden die gereinigten Kristalle einer Austauschanlage 4 über Strang 14 zugeführt, in der sie gemäss der DE-OS 2'648'982 unter Zugabe von Wasser über Strang 13 und von Anilin, das über Strang 8 in der Anlage 4 eingespeist wird, aufgearbeitet werden.

Die wiederum relativ vorzugsweise mit o-Isomeren bzw. Polyaminen angereicherte Waschflüssigkeit bzw. Restlösung gelangt über Strang 15 in eine zweite gleichartige Austauschanlage 5, der auch ein Teil der Restlösung bzw. Mutterlauge aus der Kristallisationsstufe 2 über Strang 11 zuströmt; in der Anlage 5 werden Restlösung und Waschflüssigkeit in der gleichen Weise aufgearbeitet wie das 4,4'-MDA-Salz in Anlage 4.

Bei der genannten Aufarbeitung werden das 4,4'-MDA bzw. die isomeren 2,2'- und 2,4'-MDA-Salze und/oder die Polyaminsalze von den Säureresten, an die sie chemisch gebunden sind, getrennt, und über Strang 17 bzw. 18 als Endprodukt weggeführt. Die verdünnten, durch den Austausch an Anilin gebunden Säurereste gelangen über Strang 16 in einen gemeinsamen Eindampfer 6, in dem die Anilinsalzlösungen aufkonzentriert werden, ehe sie über Strang 12 in den Kondensationsraum 1 zurückgespeist werden.

Das dabei abgedampfte Wasser verlässt die Anlage entweder über Strang 13b oder wird ebenfalls wieder zurückgeführt und über die verschiedenen Stränge 13 in die Stufe 3, 4 und/oder 5 eingespeist.

Nachstehendes Beispiel 1 zeigt bei gleichbleibendem, konstanten Anilin/Formaldehyd-Verhältnis den Einfluss des Mischungsverhältnisses der Säurekatalysatoren — an dem Beispiel von TFE und MSS — auf die Kristallisationstemperatur und die Kristallausbeute, sowie die Zusammensetzung der aus den Kondensationsprodukten erhaltenen Kristallgemische.

In drei Doppelwandgefässen, die je mit einem Thermostaten versehen sind, werden vorgelegt, um Mischungsverhältnisse zu erhalten:

| A | B | C |
|---|---|---|
| MSS/TFE 3 : 1 | MSS/TFE 1 : 3 | reine TFE |
| 93 g Anilin | 93 g Anilin | 92 g Anilin |
| 28,2 g TFE | 85,5 g TFE | 114 g TFE |
| 103 70% MSS | 34,2 g 70% MSS | 169 g H$_2$O |
| 106,3 g H$_2$O | 155,8 g H$_2$O | |

In jedem der Gefässe befinden sich dadurch homogene Lösungen mit einem Salzgehalt von 55 Gew.-% und einem Wassergehalt von 45 Gew.-%; die Gefässe werden auf einer Temperatur von 40°C gehalten.

In jeder dieser Salzlösungen ist aus Ausgangsanilin 100%ig protoniert. In jedes Gefäss werden bei der erwähnten Temperatur 20,77 g (0,25 Mol) Formaldehyd in Form einer 73%igen, wässrigen Lösung zugegeben, so dass das Mol-Verhältnis zwischen Anilin und Formaldehyd 4 : 1 beträgt. Anschliessend lässt man das Gemisch rund 30 min bei 50°C, etwa 30 min bei 70°C und ca. 1 Stunde bei 90°C reagieren.

Hierauf werden die Lösungen der bei dieser Kondensation entstandenen Produkte, die aus p- und o-Isomeren sowie Polyaminen bestehen, abgekühlt. Während der Abkühlung beginnen bei Kristallisationstemperaturen von

| A | B | C |
|---|---|---|
| ca. 27 | 38 | 50 (°C) |

Kristalle auszufallen, die abfiltriert werden, wobei sich Kristallausbeuten in Gramm von

| A | B | C |
|---|---|---|
| 97 | 109 | 111,4 (g) |

ergeben.

Eine gaschromatografische Analyse der Kristalle, nach deren Neutralisation mit NaOH — die im Labor statt dem im Zusammenhang mit der Figur geschilderten Austauschverfahren durchgeführt wird — ergibt folgende Zusammensetzung, die für alle drei Lösungen praktisch gleich ist:

96,2% 4,4'-Methylendianilin (MDA)
0,8% 2,4'-       »
3 % mehrkernige Polyamine (PA).

Während — was zu erwarten war — die Kristallausbeute mit steigendem Anteil an TFE ebenfalls zunimmt, wird der Anteil an 4,4'-MDA durch Änderungen des Mischungsverhältnisses der beiden verwendeten Säuren praktisch nicht beeinflusst.

In Beispiel 2 soll der Einfluss des Anilin/Formaldehyd-Verhältnisses bei konstanter Zusammensetzung des Säurekatalysators, d.h. konstantem Verhältnis von TFE zu MSS, auf das Verhältnis an o- zu p-Anteilen des MDA erfasst werden.

In jedem der drei Doppelwandgefässe, versehen wiederum mit einem Thermostaten, werden in diesem Beispiel vorgelegt:

| 93 g (1 Mol) | Anilin |
|---|---|
| 57 g (0,5 Mol) | TFE |
| 68,5 g (0,5 Mol) | MSS 70% |
| 141,5 g | H$_2$O |

was wiederum 55%ige wässrige homogene Salzlösungen ergibt. Zu diesen ebenfalls wieder 100% protonierten Gemischen werden wiederum bei 40°C folgende Mengen an Formaldehyd in Form einer 37%igen wässrigen Lösung zugegeben:

| A | B | C |
|---|---|---|
| 20,25 g | 28,3 g | 36,45 (g) |

Das Molverhältnis zwischen Anilin und Formaldehyd beträgt somit

| A | B | C |
|---|---|---|
| 4 : 1 | 2,85 : 1 | 2,22 : 1 |

Nach der Formaldehydzugabe werden die Lösungen behandelt wie in Beispiel 1.

Beim Abkühlen ergeben sich als Kristallisationspunkte, bei denen die ersten Kristalle sichtbar ausfallen,

| A | B | C |
|---|---|---|
| 28 | 29 | 31 (°C) |

Nach vollständigem Ausfällen erhält man als Endausbeute die folgenden Kristallausbeuten:

| A | B | C |
|---|---|---|
| 60 | 64 | 112 (g) |

Eine gleichartige Analyse der Kristalle wie bei Beispiel 1 ergibt:

| A | B | C |
|---|---|---|
| 96% 4,4'- | 95,1% 4,4'- | 94 % 4,4'-MDA |
| 1% 2,4'- | 0,9% 2,4'- | 1,1% 2,4'-MDA |
| 3% | 4,1% | 4,9% höhere PA |

Es zeigt sich also eine leichte Abnahme an 4,4'-MDA mit steigendem Formaldehyd-Anteil bei gleichzeitiger Vergrösserung des Anteils an Polyaminen.

## Patentansprüche

1. Verfahren zur selektiven Gewinnung von Methylenbrücken aufweisenden Polyarylaminen, die durch Umsetzung von Anilin und Formaldehyd in Anwesenheit von Trifluoressigsäure und/oder ihrer homologen Perfluorcarbonsäuren, gegebenenfalls im Gemisch mit anderen Säuren, deren pKa-Wert kleiner als 2,5 ist, in hochprotonierter Lösung hergestellt worden sind, dadurch gekennzeichnet, dass man das in wässriger Lösung als Gemisch aus Polyaminen und Diarylaminen vorliegende Kondensationsprodukt in einen Temperaturbereich abkühlt, in dem im wesentlichen das 4,4'-Methylendianilin als Salz auskristallisiert, während das restliche Gemisch weitgehend in Lösung bleibt.

2. Verfahren nach Anspruch 1, dadurch gekennzeichnet, dass man die Kristallisation je nach Säuremischung und Protonierungsgrad zwischen 0 und 80°C, vorzugsweise zwischen 20 und 50°C, durchführt.

3. Verfahren nach Anspruch 2, dadurch gekennzeichnet, dass der Protonierungsgrad des Anilins 60 bis 100%, vorzugsweise zwischen 95 und 100%, beträgt.

4. Verfahren nach Anspruch 2 oder 3, dadurch gekennzeichnet, dass als Säuregemisch Mischungen aus Trifluoressigsäure und Methansulfonsäure dienen.

5. Verfahren nach einem der Ansprüche 1 bis 4, dadurch gekennzeichnet, dass man die erhaltene Kristallmasse mit Wasser, gegebenenfalls unter Zusatz des Ausgangsanilins, wäscht bzw. rekristallisiert.

6. Verfahren nach einem der Ansprüche 1 bis 4, dadurch gekennzeichnet, dass man die erhaltene Kristallmasse in einer wässrigen Lösung des Anilinsalzes der Trifluoressigsäure und/oder mindestens eines ihrer Homologen bzw. der Gemische mit anderen Säuren wäscht bzw. rekristallisiert.

7. Verfahren nach einem der Ansprüche 1 bis 6, dadurch gekennzeichnet, dass man die Kristallisation und die Nachbehandlung in mehreren Stufen durchführt.

## Claims

1. A process for the selective production of methylene bridge containing polyarylamines which have been produced in highly protonized solution by the reaction of aniline and formaldehyde in the presence of trifluoroacetic acid and/or homologous perfluorocarboxylic acids thereof, if required in mixture with other acids the pK value of which is less than 2.5, characterised in that the condensation product present in aqueous solution in the form of a mixture of polyamines and diarylamines is cooled in a temperature range in which substantially 4,4'-methylene dianiline crystallizes out in the form of a salt, while the remaining mixture remains substantially in solution.

2. A process according to claim 1, characterised in that the crystallization is carried out between 0 and 80°C, preferably between 20 and 50°C, depending upon the acid mixture and degree of protonization.

3. A process according to claim 2, characterised in that the degree of protonization of the aniline is 60 to 100%, preferably between 95 and 100%.

4. A process according to claim 2 or 3, characterised in that mixtures of trifluoroacetic acid and methanesulphonic acid are used as acid mixture.

5. A process according to any one of claims 1 to 4, characterised in that the resulting crystal product is washed and recrystallized with water, with the addition of the initial aniline if required.

6. A process according to any one of claims 1 to 4, characterised in that the crystal product obtained is washed or recrystallized in an aqueous solution of the aniline salt of trifluoroacetic acid and/or at least one of the homologues thereof or of the mixture with other acids.

7. A process according to any one of claims 1 to 6, characterised in that the crystallization and subsequent treatment are carried out in a number of stages.

## Revendications

1. Procédé d'obtention sélective de polyarylamines présentant des ponts méthyléniques, qui ont été produites en solution fortement protonée par réaction d'aniline et de formaldéhyde en présence d'acide trifluoracétique et/ou de ses homologues perfluorocarboxyliques, éventuellement en mélange avec d'autres acides dont le pKa a une valeur inférieure à 2,5, caractérisé en ce qu'on refroidit le produit de condensation se présentant en solution aqueuse sous la forme d'un mélange de polyamines et de diarylamines dans une plage de températures dans laquelle, principalement, la 4,4'-méthylènedianiline cristallise sous forme de sel, tandis que le mélange résiduel reste largement en solution.

2. Procédé suivant la revendication 1, caractérisé en ce que la cristallisation est conduite entre 0 et 80°C, de préférence entre 20 et 50°C selon le mélange d'acides et le degré de protonation.

3. Procédé suivant la revendication 2, caractérisé en ce que le degré de protonation de l'aniline s'élève à 60-100%, de préférence à 95-100%.

4. Procédé suivant la revendication 2 ou 3, caractérisé en ce que des mélanges d'acide trifluoracétique et d'acide méthanesulfonique sont utilisés comme mélange d'acides.

5. Procédé suivant l'une des revendications 1 à 4, caractérisé en ce qu'on lave et fait recristalliser la masse cristalline obtenue avec de l'eau, le cas échéant additionnée de l'aniline de départ.

6. Procédé suivant l'une des revendications 1 à 4, caractérisé en ce qu'on lave et fait recristalliser la masse cristalline obtenue dans une solution aqueuse du sel d'aniline de l'acide trifluoracétique et/ou d'au moins l'un de ses homologues ou de leurs mélanges avec d'autres acides.

7. Procédé suivant l'une des revendications 1 à 6, caractérisé en ce qu'on conduit la cristallisation et le traitement subséquent en plusieurs étapes.

0 009 094